# FASCICULE DE BREVET EUROPEEN

(11) **EP 1 341 809 B1**
(45) Date de publication et mention de la délivrance du brevet: **02.11.2006**
(21) Numéro de dépôt: 01270549.7
(22) Date de dépôt: 14.12.2001
(51) Int. Cl.: C07K 14/035

(54) **PEPTIDE DE LA GLYCOPROTEINE B DE L'HERPESVIRUS HUMAIN 7 UTILISABLE NOTAMMENT DANS UN TEST SEROLOGIQUE ELISA**
PEPTIDE AUS DEM B-GLYCOPROTEIN DES MENSCHLICHEN HERPESVIRUS-7, BESONDERS ANWENDBAR BEI SEROLOGISCHEN ELISA-NACHWEISEN
GLYCOPROTEIN PEPTIDE OF THE HUMAN HERPESVIRUS-7 FOR USE IN PARTICULAR IN AN ELISA SEROLOGIC TEST

(30) Priorité: 15.12.2000 FR 0016431
(43) Date de publication de la demande: 10.09.2003
(73) Titulaire: UNIVERSITE PIERRE ET MARIE CURIE (PARIS VI), 75252 Paris Cédex 05 (FR)
(72) Inventeur: AGUT, Henri, F-75016 Paris (FR); FRANTI, Michael, F-92250 La Garenne-Colombes (FR)
(74) Mandataire: Desaix, Anne
(86) Numéro de dépôt international: PCT/FR2001/004010
(87) Numéro de publication internationale: WO 2002/048184

(56) Documents cités:
- WO-A-99/02554
- O MARIN ET AL.: "Synthetic peptids including acidic clusters as substrates of yeast casein kinase-2" INTERNATIONAL JOURNAL OF PEPTIDE AND PROTEIN RESEARCH., vol. 36, 1990, pages 374-380, XP002181103 MUNKSGAARD, COPENHAGEN., DK ISSN: 0367-8377
- M FRANTI ET AL.: "Preferential associations of alleles of three distinct genes argue for the existence of two prototype variants of human herpesvirus 7" JOURNAL OF VIROLOGY., vol. 73, no. 11, novembre 1999 (1999-11), pages 9655-9658, XP002181104 ICAN SOCIETY FOR MICROBIOLOGY US
- M FRANTI ET AL.: "Definition and distribution of glycoprotein B gene alleles of human herpesvirus 7" JOURNAL OF VIROLOGY., vol. 72, no. 11, 1998, pages 8725-8730, XP002181105 THE AMERICAN SOCIETY FOR MICROBIOLOGY., US ISSN: 0022-538X
- DATABASE CA [en ligne] CHEMICAL ABSTRACTS SERVICE, COLUMBUS, OHIO, US; HATA, ATSUKO ET AL: "Identification and analyses of glycoprotein B of human herpesvirus 7" retrieved from STN Database accession no. 126:128384 CA XP002181106 & VIRUS RES. (1996), 46(1,2), 125-137, 1996,

## Description

La présente invention a trait à des peptides de la glycoprotéine B de l'herpesvirus humain 7 (HHV-7), utilisables pour réaliser un test sérologique spécifique de HHV-7, ou dans des compositions pharmaceutiques destinées à stimuler une réponse immunitaire dirigée contre HHV-7.

L'herpesvirus humain 7 est un β-herpesvirus (virus enveloppé à ADN), découvert en 1990. Ce virus est très largement répandu dans la population générale, donne une primo-infection précocement dans la vie et, comme les autres herpesvirus, se maintient indéfiniment sous forme latente dans l'organisme infecté. Le HHV-7 est proche génétiquement du cytomégalovirus (CMV) et de l'herpesvirus humain 6 (HHV-6) qui, surtout dans le cas du CMV, sont des virus pathogènes majeurs. La responsabilité du HHV-7 dans des maladies humaines paraît modeste dans l'état actuel des connaissances mais son pouvoir pathogène est loin d'avoir été complètement exploré. On peut craindre que, lors d'une immunodépression, ce pouvoir pathogène soit exacerbé et donne lieu à des infections opportunistes graves, comme les autres herpesvirus. En particulier, ceci peut être le cas après une transplantation d'organe. La réactivation de HHV-7 pourrait être pathogène et notamment aggraver l'état d'immunodépression, du fait du tropisme sélectif de HHV-7 pour les lymphocytes T CD4+.

Par ailleurs, les molécules actives contre le CMV et déjà largement utilisées en thérapeutique médicale (foscarnet, cidofovir, ganciclovir) sont actives contre le HHV-7. L'existence *a priori* de traitements actifs souligne encore davantage l'intérêt de poursuivre des études sur la détection d'infections par ce virus.

Il n'existe actuellement sur le marché aucun moyen complètement satisfaisant pour diagnostiquer de façon spécifique une infection chronique par HHV-7. Des méthodes de diagnostic spécifique d'une infection par HHV-7 basées sur l'amplification d'une portion du génome viral par PCR, en utilisant des amorces spécifiques de HHV-7 ont été décrites, par exemple dans WO 9703345A. Cependant, de telles méthodes manquent de sensibilité pour le diagnostic d'une infection chronique, et sont davantage destinées à détecter (et éventuellement quantifier) une infection active.

La détection d'une infection chronique nécessite d'effectuer un test sérologique, qui révèlera la présence ou non d'anticorps anti-HHV-7 dans l'échantillon. Les systèmes de sérodiagnostic de HHV-7 commercialisés actuellement ne sont pas complètement satisfaisants. En effet, le diagnostic de HHV-7 est aujourd'hui réalisé en utilisant comme antigène des cellules infectées par HHV-7. Cependant, la proximité génétique des autres β-herpesvirus, notamment de HHV-6, nuit à la spécificité de ce type de test. Des étapes d'adsorption sur des antigènes de cellules infectées par les autres virus sont nécessaires pour augmenter la spécificité de ces tests.

La détermination de molécules antigéniques réellement spécifiques de HHV-7 est donc une avancée significative dans la mise au point d'un test de sérodiagnostic spécifique de ce virus et facile à réaliser.

Dans cette optique, Secchiero *et al*. ont décrit des antigènes d'une protéine de HHV-7, la pp85 (WO 99/02554). Certains de ces antigènes semblent spécifiques de HHV-7.

Cependant, la protéine pp85 codée par le gène U14 est une phosphoprotéine du tégument pour laquelle on peut s'attendre à une spécificité moindre que celle portée par les glycoprotéines de l'enveloppe virale. De plus, bien qu'un épitope spécifique ait été localisé dans la partie C-terminale de la protéine, les peptides portant cet épitope n'ont pu être utilisés pour établir un test ELISA fonctionnel (Stefan *et al*., J Clin Microbiol 1999 ; 37 :3980-5), ce qui reste l'objectif prioritaire en sérologie du HHV-7. Enfin, en termes de protection immune, il est reconnu classiquement que les glycoprotéines de l'enveloppe virale, et en particulier la gB, sont une meilleure cible pour les anticorps neutralisants que les protéines du tégument.

**Marin et al. Int. J. Peptide Protein Res. 36, 374-38, décrit des peptides synthétiques qui ont été préparés pour examiner le site spécifique pour le casein-Kinase. Un peptide comporte la séquence**

**4 - Arg - Ser - Glu - Glu - Glu - Glu - Glu - OH,**

La présente invention porte précisément sur des peptides de la glycoprotéine B (gB) de HHV-7.

La gB du HHV-7, présente à la surface des cellules infectées et des virions, participe à l'attachement et à la fusion du virus à la surface des cellules. Par analogie avec les autres herpesvirus, il a été supposé que la gB est immunogène, suscitant des anticorps neutralisants et une réponse immunitaire cellulaire chez les sujets infectés. Tous les résultats publiés jusqu'à présent corroborent cette hypothèse, même si la gB, protéine indispensable du virus, n'apparaît pas comme la protéine immunodominante en termes de réactivité des anticorps sériques humains dans les tests d'immunoblot actuellement utilisés.

**Hata et al. (XP - 002181106) décrit la structure entière de la glycoprotéine B - (gB) de HHV-7.**

La première partie de l'étude de la gB a consisté à amplifier le gène de la gB par la réaction d'amplification en chaîne par polymérase (PCR) et analyser les produits de PCR correspondants chez plus de 100 sujets. Cette étude a montré une très grande stabilité du gène de la gB (Franti *et al*., 1998 et 1999). Cette donnée est en accord avec la comparaison de deux souches distinctes de HHV-7 dont la séquence complète a été publiée (Nicholas, 1996 ; Megaw *et al*., 1998): la divergence nucléotidique entre les deux souches est de l'ordre de 0,1%. Cependant, la détermination de la séquence nucléotidique partielle de la gB chez de nombreux individus a permis de mettre en évidence cinq sites critiques de substitution de bases, sans modification de séquence polypeptidique et de décrire 6 combinaisons distinctes de ces séquences, dénommées *allèles.* Ces allèles sont très intéressants en tant que marqueurs épidémiologiques, mais leur description ne remet pas en cause la parfaite conservation de la gB en tant que protéine virale parmi toutes les souches testées. Cette propriété en fait une bonne candidate pour définir des réactifs antigéniques susceptibles de réagir avec des anticorps dirigés contre n'importe quelle souche virale HHV-7 en circulation.

La présente invention résulte du clonage du gène entier de la gB et de son expression dans un système d'expression procaryote, le colibacille. Par ailleurs, le gène entier ainsi que la partie N-terminale ont été clonés et exprimés dans un système d'expression eucaryote, le baculovirus. Une expression stable a été obtenue dans les bactéries, ainsi qu'une expression stable avec clivage de la protéine précurseur dans le système baculovirus. L'expression à la surface des cellules d'insecte SF21 infectées par les baculovirus recombinants semble proche de celle de la gB naturelle. Cette production de protéine recombinante a permis, après transfert de la protéine sur une membrane de nitrocellulose, de tester la réactivité d'une batterie de huit sérums humains différents séropositifs pour le HHV-7, grâce à la technique de *Western blot.*

Une première série d'expériences a permis d'arriver aux conclusions suivantes : (i) il existe des anticorps spécifiques dirigés contre la gB recombinante dans les huit sérums testés, (ii) la réactivité des anticorps dirigés contre cette glycoprotéine de surface est dirigée contre la partie N-terminale de la gB qui donne le même profil de réaction que la protéine entière. Ceci confirme l'hypothèse que la gB induit des anticorps chez les sujets infectés et que des formes partielles de gB recombinante permettent de détecter ces anticorps. Les résultats sont cependant entachés par la présence de bandes parasites, probablement en relation avec des phénomènes de réactivité non spécifique, et la technique de *Western blot* est peu adaptée à une utilisation à grande échelle.

Afin de remédier à ces problèmes, les auteurs de la présente invention ont entrepris de définir et faire synthétiser un peptide localisé dans la région N-terminale de la gB. Ce peptide devrait présenter les deux caractéristiques suivantes : (i) peptide dérivé d'une région très hydrophile de la gB, susceptible d'interagir avec un anticorps ; (ii) peptide présentant une très faible homologie avec les gB des deux autres β-herpesvirus humains, le HHV-6 (qui comprend deux variants, HHV-6A et HHV-6B) et le CMV, afin d'éviter les réactions sérologiques croisées. Le choix s'est porté sur un peptide de 24 acides aminés, correspondant aux acides aminés 129-152 de la gB, localisé dans la région N-terminale et défini par la séquence peptidique :

Afin de préciser et d'identifier la sélection de ce peptide dans la protéine, on pourra se référer aux figures 1 à 3.
La Figure 1 représente les courbes d'hydrophilicité de la gB de HHV-7 (1A), HHV-6A (1B), HHV-6B (1C), et CMV (1D), sur lesquelles la région correspondant à la région exposée de la gB de HHV-7 est localisée par une flèche et le peptide H7GB 129-152 figuré par un trait noir.
La Figure 2 représente une étude plus fine de l'hydrophilicité de la région choisie, le peptide H7GB 129-152 étant figuré par le trait noir.
La Figure 3 montre l'alignement des séquences protéiques de la gB de HHV-7, HHV-6A, HHV-6B et CMV, dans la région du peptide H7GB 129-152 (représenté sur la première ligne).

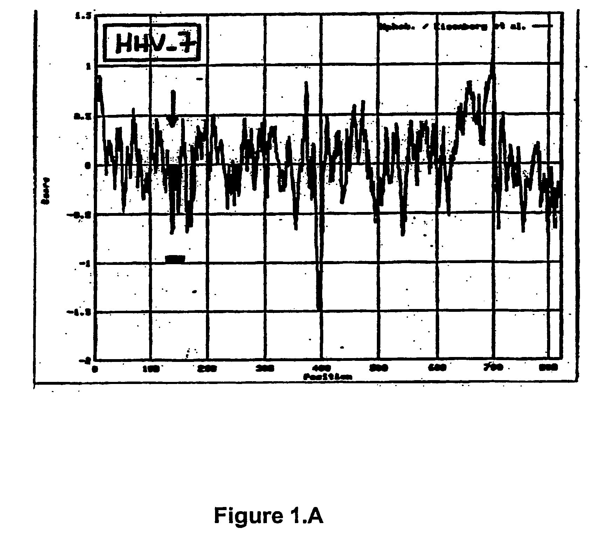

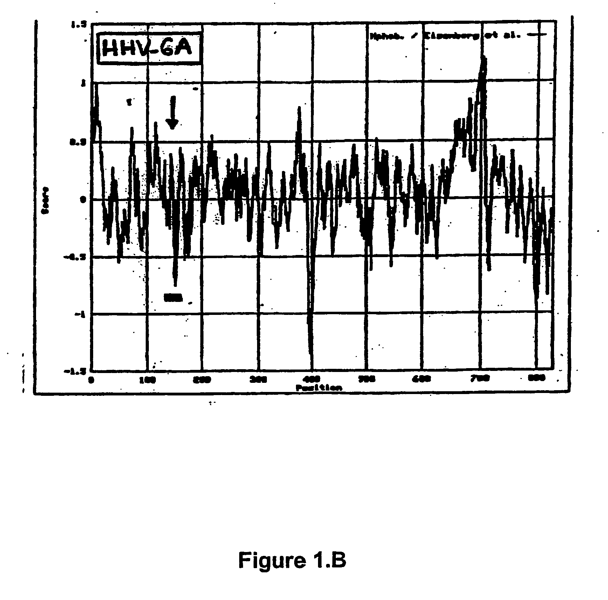

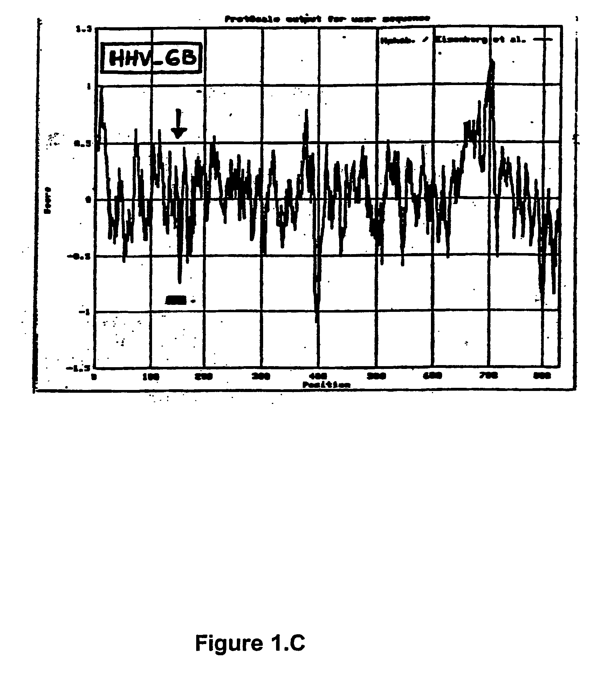

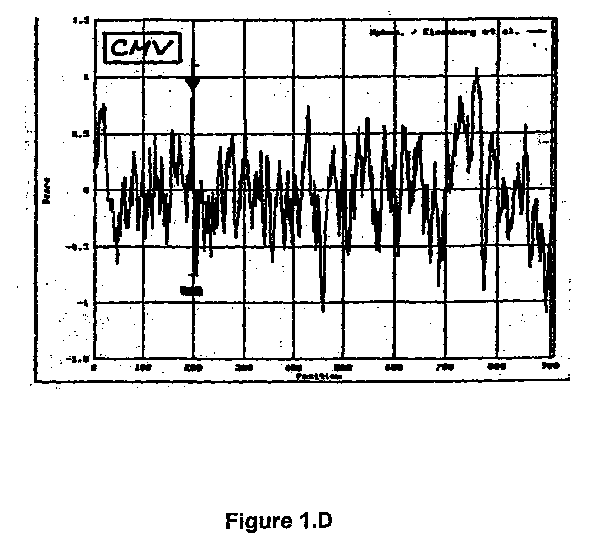

Cette région de 24 acides aminés offre l'avantage d'être dans le quart N-terminal de la protéine et d'être *a priori* bien exposée à l'extérieur de la molécule du fait de son hydrophilicité. Cette région exposée est retrouvée en position sensiblement homologue pour les autres β-herpesvirus (HHV-6A, HHV-6B, CMV) (Figure 1).

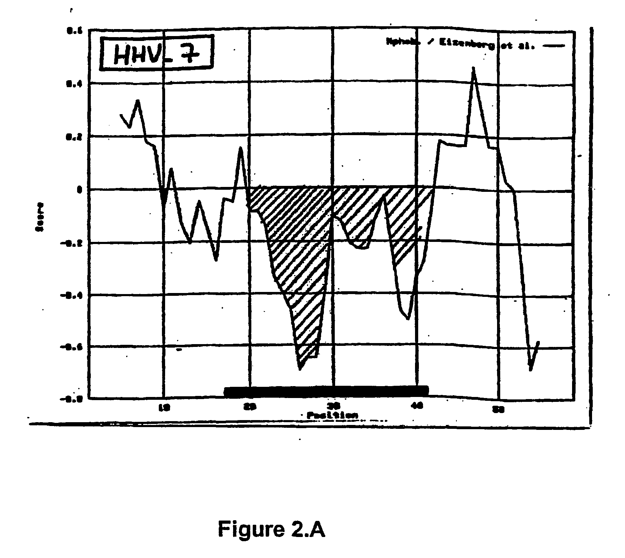

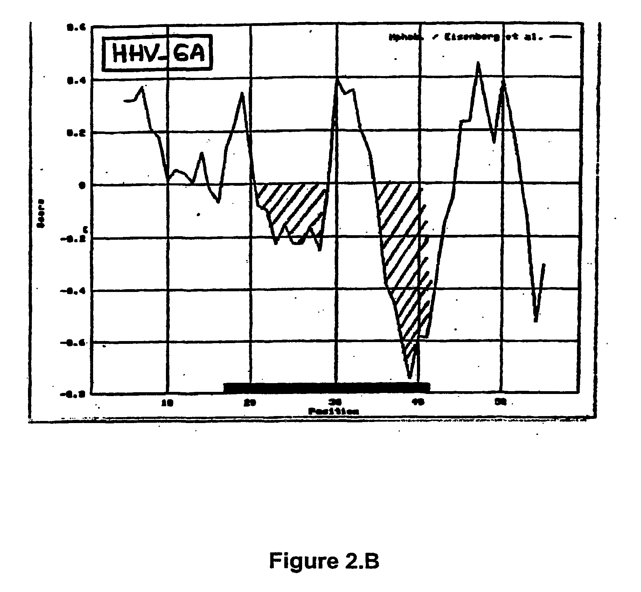

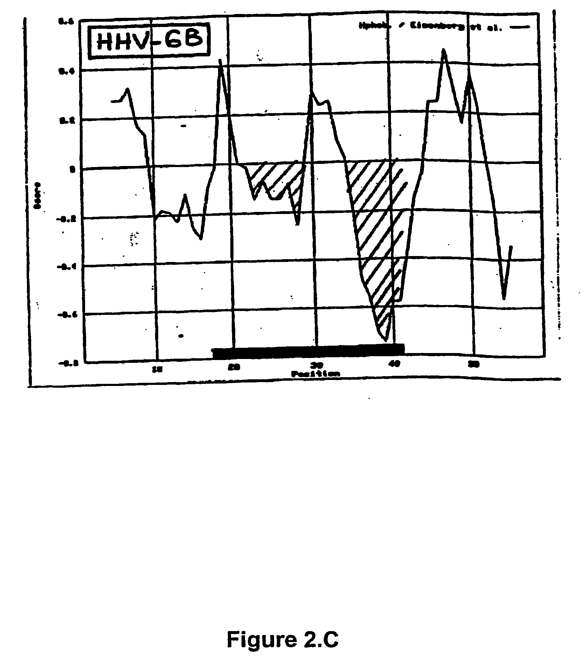

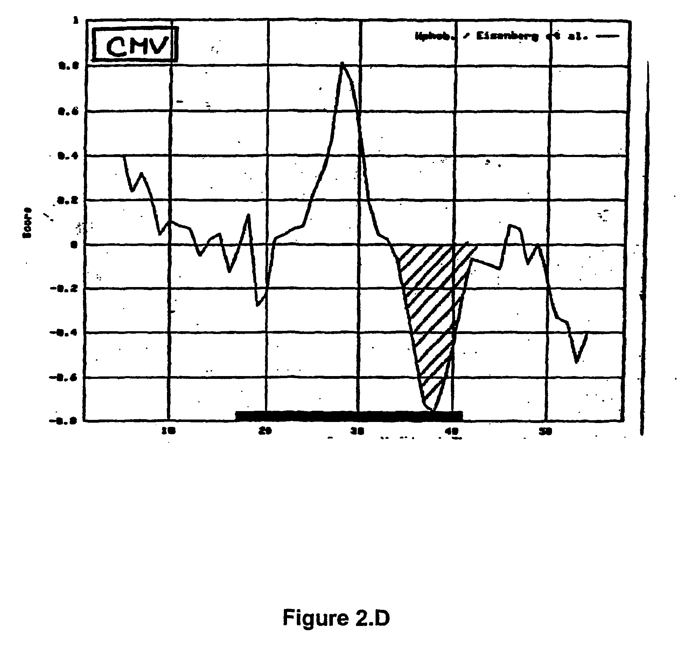

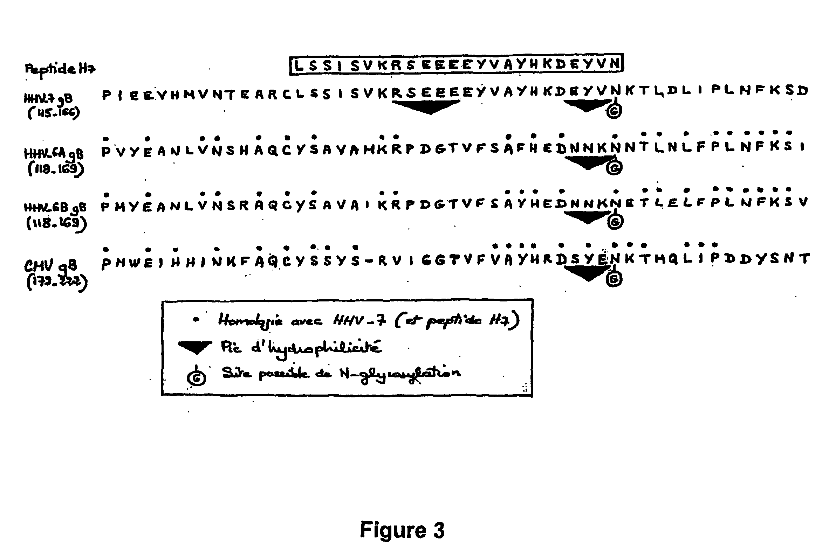

Une étude plus fine de l'hydrophilicité de la région choisie montre cependant, après alignement des séquences, que le pic d'hydrophilicité du HHV-7 n'occupe pas exactement la même position que ceux des HHV-6A, HHV-6B, et CMV (Figure 2). L'étude de la séquence protéique, toujours après alignement (Figure 3) confirme que le HHV-7 présente dans la région choisie pour l'utilisation d'un peptide une séquence originale très hydrophile centrée sur les résidus 136-141 :

Arg-Ser-Glu-Glu-Glu-Glu (RSEEEE)

**dans la préparation d'une composition pour la détection sérologique de HHV-7, le diagnostic de HHV-7 chez un sujet, ou la stimulation d'une réponse immunitaire dirigée contre HHV-7 chez un sujet.**

Cette séquence n'est pas retrouvée pour les autres β-herpesvirus. De façon à ce que ce motif, qui représente vraisemblablement tout ou partie de l'épitope ou des épitopes dominant(s), soit présenté de façon correcte après adsorption sur un support solide, les séquences bordantes ont été adjointes à cette séquence particulière pour aboutir à la séquence totale du peptide H7GB 129-152 ci-dessus.

Il faut noter que ce peptide contient, à son extrémité C-terminale, un motif Asp-Glu-Tyr-Val-Asn, *a priori* très hydrophile qui pourrait aussi se comporter comme un épitope vis-à-vis des anticorps anti-gB. Cependant, il est peu probable que cette région à l'extrémité libre du peptide adopte la bonne conformation pour se comporter en épitope linéaire. Par ailleurs, le résidu terminal Asn peut être un site de N-glycosylation de la gB, puisqu'il appartient à une séquence canonique Asn-X-Thr, bien conservée pour les quatre β-herpesvirus. L'encombrement stérique engendré par la glycosylation (si elle existe) s'opposerait probablement à l'exposition du motif Asp-Glu-Tyr-Val-Asn comme épitope à la surface de la molécule.

La présente invention porte ainsi sur des peptides immunogènes consistant en 15 à 25 acides aminés et comportant la séquence Ser - Val - Lys - Arg - Ser - Glu - Glu - Glu - Glu - Tyr - Val - Ala, le cas échéant modifiée par sutstitution(s) conservative(sà qui ne modifie(nt) pas la réactivité immunologique dudit peptide d'une région hydrophile de la glycoprotéine B (gB) de l'herpèsvirus humain 7 (HHV-7), et réagissant de manière spécifique avec des anticorps dirigés contre HHV-7. Par « réagissant de manière spécifique avec des anticorps dirigés contre HHV-7 », on entend que des anticorps dirigés contre des β-herpesvirus autres que HHV-7 (par exemple, contre HHV-6) n'ont pas d'affinité particulière pour ces peptides.

De manière préférée, la séquence d'acides aminés de la gB présente dans les peptides de l'invention provient de la région N-terminale de cette protéine.

Les modification éventuelles de cette séquence seront effectuées de telle façon que la présentation du motif Arg-Ser-Glu-Glu-Glu-Glu permette toujours son interaction avec des anticorps anti-HHV-7. Il peut en particulier s'agir de substitution d'acides aminés par des acides aminés d'hydrophilicité analogue.

De manière préférée mais non limitative, les peptides de l'invention comportent 15 à 25 acides aminés.

Un peptide particulier de l'invention est le peptide H7GB 129-152, correspondant aux acides aminés 129 à 152 de la gB et défini par la séquence peptidique : Leu-Ser-Ser-Ile-Ser-Val-Lys-Arg-Ser-Glu-Glu-Glu-Glu-Tyr-Val-Ala-Tyr-His-Lys-Asp-Glu-Tyr-Val-Asn. Le cas échéant, la séquence de ce peptide peut être modifiée par une ou plusieurs modifications conservatives d'acides aminés, à condition que la réactivité immunologique de ce peptide ne soit pas modifiée.

Les peptides de l'invention tels que décrits ci-dessus peuvent en outre être modifiés par l'ajout, à une de leurs extrémités ou aux deux, de un ou plusieurs acides aminés terminaux hétérologues à la gB. Le cas échéant, ces peptides peuvent aussi être marqués.

Les huit sérums humains testés précédemment en *Western blot* ont été utilisés pour mettre au point les meilleures conditions de réactivité de ce peptide dans un système ELISA (exemple 1 ci-après). Ont été ainsi définis (en densité optique) :
- la valeur moyenne du bruit de fond de la réaction : 0,09 ;
- la valeur moyenne des résultats obtenus avec les sérums négatifs : 0,18 ;
- un seuil de positivité de 0,23, correspondant à la moyenne des négatifs augmentée de 2 écarts-types (risque d'erreur de 5%) ou de 0,25 correspondant à la moyenne des négatifs augmentée de 3 écarts-types (risque d'erreur inférieur à 1%) ;
- un spectre de valeurs des sérums positifs allant environ de 0,4 à 1,1.

La reproductibilité des résultats s'est révélée très satisfaisante, aussi bien en comparant des expériences séparées menées simultanément (étude de la variabilité intra-essai) que des expériences séparées menées à des jours différents (étude de la variabilité inter-essais). La spécificité des résultats vis-à-vis du peptide utilisé a été vérifiée de plusieurs façons :
- test des sérums HHV-7 positifs vis-à-vis d'un autre peptide viral non pertinent (peptide de HHV-8) dans les mêmes conditions ou en l'absence de tout peptide fixé dans les puits de réaction : les résultats sont constamment sous le seuil de positivité (exemple 2 ci-après) ;
- test des sérums HHV-7 positifs vis-à-vis du peptide HHV-7 après adsorption des sérums sur différents extraits de cellules infectées : le signal de positivité est significativement diminué, voire aboli quand des cellules sont infectées par le HHV-7 alors qu'il est conservé ou peu modifié quand les cellules sont infectées par le CMV et le HHV-6 (exemple 3 ci-après) ;
- absence de réactivité avec six sérums HHV-7 séronégatifs(exemple 4 ci-après) ;
- action inhibitrice du peptide soluble, mis en contact préalablement avec les sérums, sur la réactivité ELISA par un phénomène de compétition dose-dépendant (exemple 5 ci-après).

En particulier, un peptide portant les résidus Arg-Ser-Glu-Glu-Glu-Glu, entourés d'acides aminés partiellement ou totalement hétérologues à la gB, est susceptible de réagir spécifiquement avec des anticorps dirigés contre HHV-7, à condition qu'il soit correctement présenté. En effet, l'influence de l'insertion ou la délétion de résidus entourant le motif Arg-Ser-Glu-Glu-Glu-Glu, sur les propriétés immunologiques du peptide ainsi obtenu sont difficiles à prévoir si la charge totale du peptide et la polarité des résidus modifiés sont globalement conservés. Dans tous les cas, seuls la synthèse du nouveau peptide et son test (par exemple, par ELISA tel que décrit dans l'exemple 1), permettront de mesurer exactement le retentissement fonctionnel des modifications.

Les propriétés des peptides de l'invention leur permettent donc de servir de base pour le développement d'un test sérologique spécifique dont les indications seraient, entre autres :
- le suivi et l'étude des infections à HHV-7 chez les enfants au moment de la primo-infection (1-3 ans) ;
- le suivi sérologique des sujets immunodéprimés avec l'idée de corréler les modifications de réactivité sérologique au degré de dysfonctionnement du système immunitaire ;
- la mise en évidence d'une corrélation entre le titre des anticorps anti-gB et la capacité de contrôle de l'infection à HHV-7 ;
- une claire distinction entre les infections à HHV-6 et HHV-7 devant certaines manifestations cliniques particulières rattachées à la primo-infection et dans les études de séroprévalence générale ;
- la détermination de la sérologie HHV-7 d'un candidat à une greffe d'organe et d'un donneur d'organes potentiellement utilisables pour des greffes.

La présente invention porte aussi sur un kit ou trousse permettant le diagnostic spécifique du HHV-7, comportant un peptide tel que ceux décrits ci-dessus. Le cas échéant, ce peptide peut être fixé sur un support. De manière préférée, un kit de diagnostic selon l'invention comporte en outre un anticorps détectable, dirigé contre une ou plusieurs classes d'immunoglobulines humaines. Cet anticorps détectable peut être radio-marqué, fluorescent, ou lié à une enzyme. Dans le cas ou l'anticorps détectable est lié à une enzyme, le kit comprend avantageusement un substrat de ladite enzyme, permettant d'obtenir un résultat détectable visuellement.

Les peptides de l'invention peuvent aussi entrer dans la composition de kits de diagnostic plus complexes, permettant, outre la détection spécifique d'une infection par HHV-7, le sérodiagnostic d'un échantillon en ce qui concerne un ou plusieurs autres β-herpesvirus.

L'invention porte également sur un procédé de détection sérologique de HHV-7 dans un échantillon biologique, comportant une étape de mise en contact dudit échantillon biologique avec un peptide tel que ceux décrits ci-dessus, et une étape de détermination de la liaison du peptide avec des anticorps éventuellement présents dans l'échantillon. Dans un tel procédé, l'étape de détermination de la liaison du peptide avec des anticorps éventuellement présents dans l'échantillon peut être réalisée par toute méthode accessible à l'homme du métier permettant d'identifier une liaison spécifique entre un antigène et un anticorps, notamment par un test ELISA, par chimioluminescence, par fixation sur un support de type micro ou macro-réseau, par immunofluorescence, par radio-immuno-marquage, par agglutination ou par hémagglutination.

Par ailleurs, les propriétés immunologiques des peptides décrits ci-dessus en font des candidats potentiellement intéressants pour entrer dans la composition de préparations immunogènes destinées à favoriser une réponse immunitaire contre HHV-7 chez un sujet. Ces préparations peuvent être complexes et comporter d'autres antigènes, notamment des antigènes majeurs de HHV-7 ou éventuellement d'autres β-herpesvirus. L'invention porte donc également sur une préparation immunogène destinée à favoriser une réponse immunitaire contre HHV-7, caractérisée en ce qu'elle comporte au moins un peptide dont une région au moins provient de la gB, tel que ceux décrits ci-dessus.

Enfin, l'invention porte sur l'utilisation d'un peptide de l'invention, dans la préparation d'une composition pharmaceutique pour la détection sérologique de HHV-7, le diagnostic de HHV-7 chez un sujet, ou la stimulation d'une réponse immunitaire dirigée contre HHV-7 chez un sujet.

Les exemples et figures présentés ci-dessous à titre non limitatif, permettront de mettre en évidence certains avantages et caractéristiques de la présente invention.

### Exemple 1 : mise au point de conditions optimales de réactivité du peptide H7GB 129-152 dans un système ELISA :

Diverses concentrations de peptide (allant de 0,1 à 5 µg/ml) ont été utilisées lors de sa fixation au fond des cupules de réaction ainsi que différentes dilutions de sérum humain lors du test ELISA. Les conditions jugées comme les meilleures ont été une concentration de peptide de 0,5 µg/ml et une dilution de sérum à 1/100. Cependant, d'autres conditions, incluant des modifications des autres réactifs, pourraient conduire éventuellement à des tests plus performants.

### Exemple 2 : test des sérums HHV-7 positifs vis-à-vis d'un autre peptide viral non pertinent (peptide de HHV-8) dans un système ELISA avec le peptide H7GB 129-152 :

Huit sérums de référence séropositifs pour le HHV-7 et notés de P1 à P8 ont été testés en l'absence de peptide et vis-à-vis d'un peptide de HHV-8 ne présentant pas d'homologie avec H7GB 129-152. Les résultats (résumés dans le tableau ci-après) de la mesure de densité optique (DO) indiquent une absence de réactivité significative dans ces conditons (seuil de positivité de la réaction correspondant à une DO de 0,25).

| | DO en fonction du sérum testé | | | | | | | |
|---|---|---|---|---|---|---|---|---|
| Peptide utilisé pour l'ELISA | P1 | P2 | P3 | P4 | P5 | P6 | P7 | P8 |
| Pas de peptide | 0,05 | 0,08 | 0,07 | 0,06 | 0,06 | 0,06 | 0,05 | 0,04 |
| Peptide HHV-8 | 0,13 | 0,16 | 0,17 | 0,15 | 0,15 | 0,14 | 0,16 | 0,14 |
| H7GB 129-152 | 1,11 | 0,56 | 0,96 | 0,60 | 0,69 | 1,01 | 0,64 | 0,88 |

### Exemple 3 : test dans un système ELISA avec le peptide H7GB 129-152 de sérums HHV-7 positifs vis-à-vis du peptide H7GB 129-152, après adsorption des sérums sur différents extraits de cellules infectées :

Les huit sérums humains notés de P1 à P8 ont été adsorbés avec différents extraits de cellules infectées ou non avant d'être testés en ELISA vis-à-vis du peptide H7GB 129-152 dans les conditions préétablies. Les résultats ont été exprimés en pourcentage de réduction par rapport au signal obtenu en l'absence de toute préadsorption (voir exemple 2). Les résultats résumés ci-après indiquent que seule la préadsorption avec des cellules exprimant la gB de HHV-7 est susceptible de réduire significativement le signal.

| Préadsorption | | Pourcentage de réduction de la DO (%) | | | | | | | |
|---|---|---|---|---|---|---|---|---|---|
| Cellules | Infection | P1 | P2 | P3 | P4 | P5 | P6 | P7 | P8 |
| SupT1 | non | 5 | 29 | 0 | 11 | 20 | 0 | 11 | 17 |
| SupT1 | HHV-7 | 40 | 58 | 56 | 52 | 50 | 43 | 40 | 56 |
| Sf21 | non | 11 | 22 | 0 | 0 | 17 | 0 | 0 | 0 |
| Sf21 | Baculovirus exprimant gB HHV-7 | 46 | 66 | 30 | 26 | 57 | 26 | 60 | 42 |
| MT4 | non | 20 | 2 | 4 | 10 | 13 | 7 | 11 | 0 |
| MT4 | HHV-6 | 11 | 0 | 11 | 3 | 19 | 6 | 0 | 0 |
| FH | non | 18 | 0 | 13 | 0 | 9 | 13 | 0 | 6 |
| FH | CMV | 20 | 5 | 8 | 0 | 11 | 2 | 3 | 0 |

### Exemple 4 : test dans un système ELISA avec le peptide H7GB 129-152 de six sérums HHV-7 séronégatifs :

Six sérums de référence désignés de N1 à N6 séronégatifs pour le HHV-7 mais ayant un titre très élevé d'anticorps anti-HHV-6 (> 1280) ont été testés. Les résultats montrent une faible réactivité vis-à-vis du peptide. Ils montrent aussi, comme pour les sérums séropositifs, une variabilité, exprimée sous la forme d'un coefficient de variabilité (CV), qui est suffisamment restreinte pour envisager le développement d'un test ELISA de routine.

| | | | Coefficient de variabilité (%) | |
|---|---|---|---|---|
| Sérum testé | DO moyenne | Ecart-Type | Intra-essais | Inter-essais |
| N1 | 0.20 | 0.02 | 9,5 | 8,8 |
| N2 | 0.16 | 0.02 | 8,3 | 13,8 |
| N3 | 0.18 | 0.01 | 3,3 | 3,6 |
| N4 | 0.17 | 0.02 | 11,0 | 11,0 |
| N5 | 0.18 | 0.01 | 13,2 | 4,8 |
| N6 | 0.16 | 0.07 | 44,7 | 47,0 |

### Exemple 5 : action inhibitrice du peptide soluble, mis en contact préalablement avec les sérums, sur la réactivité ELISA :

Les sérums positifs notés de P1 à P8 ont été préincubés en présence de H7GB129-152 soluble à deux concentrations différentes puis testés en ELISA. La réduction du signal par rapport aux mêmes sérums non incubés traduit la compétition exercée par le peptide soluble vis-à-vis des anticorps anti-HHV-7.

| | Pourcentage de réduction de la DO (%) | | | | | | | |
|---|---|---|---|---|---|---|---|---|
| Concentration de H7GB 129-152 pendant la préincubation (µg/ml) | P1 | P2 | P3 | P4 | P5 | P6 | P7 | P8 |
| 5 | 27 | 30 | 32 | 48 | 14 | 23 | 37 | 24 |
| 50 | 75 | 45 | 66 | 61 | 70 | 67 | 63 | 61 |

### Références citées :

Franti M, Aubin JT, Poirel L, Gautheret-Dejean A, Candotti D, Huraux JM et Agut H . Definition and distribution analysis of glycoprotein B gene alleles of human herpes virus 7. J Virol 1998; 72:8725-30.
Franti M, Aubin JT, Gautheret-Dejean A, Malet I, Cahour A, Hufaux JM et Agut H. Preferential associations of alleles of three distinct genes argue for the existence of two prototype variants of human herpesvirus 7. J Virol 1999, 73:9655-58.
Nicholas J. Determination and analysis of the complete nucleotide sequence of human herpesvirus 7. J Virol 1996; 70:5975-89.
Megaw AG, Rapaport D, Avidor B, Frenkel N, Davison AJ. The DNA sequence of the RK stain of human herpesvirus 7. Virology 1998; 244:119-32.

## Revendications

1. Peptide immunogène, **caractérisé en ce qu'**il consiste en 15 à 25 acides aminés et **en ce qu'**il comporte la séquence
Ser-Val-Lys-Arg-Ser-Glu-Glu-Glu-Glu-Tyr-Val-Ala,
le cas échéant modifiée par substitution(s) conservative(s) qui ne modifie(nt) pas la réactivité immunologique dudit peptide, et qui conserve la séquence Arg-Ser-Glu-Glu-Glu-Glu.

2. Peptide selon la revendication 1, correspondant aux acides amines 129 a 152 de la gB et défini par le séquence peptidique :
Leu-Ser-Ser-Ile-Ser-Val-Lys-Arg-Ser-Glu-Glu-Glu-Glu-Tyr-Val-Ala-Tyr-His-Lys-Asp-Glu-Tyr-Val-Asn,
le cas échéant modifiée par substitution(s) conservative(s) qui ne modifie (nt) pas la réactivité immunologique dudit peptide, et qui conserve la séquence Arg-Ser-Glu-Glu-Glu-Glu.

3. Peptide selon la revendication 1 ou 2, comportant en outre un ou plusieurs acides aminés terminaux hétérologues à la gB.

4. Kit permettant la diagnostic spécifique du HHV-7, comportant un peptide selon l'une quelconque des revendications 1 à 3.

5. Kit de diagnostic selon la revendication 4, **caractérisée en ce que** ledit peptide est fixé sur un support.

6. Kit de diagnostic selon la revendication 4 ou 5, comportant en outre un anticorps détectable dirigé contre une ou plusieurs classes d'immunoglobulines humaines.

7. Kit de diagnostic selon la revendication 6, dans lequel l'anticorps détectable est radio-marqué, fluorescent ou lié à une enzyme.

8. Kit de diagnostic selon la revendication 7, dans lequel l'anticorps détectable est lié à une enzyme et comportant en outre un substrat de ladite enzyme permettant d'obtenir un résultat détectable visuellement.

9. Kit de diagnostic selon l'une quelconque des revendications 4 à 8, permettant en outre le diagnostic sérologique d'un ou plusieurs autres betaherpesvirus.

10. Procédé de détection sérologique de HHV-7 dans un échantillon biologique, comportant une étape de mise en contact dudit échantillon biologique avec un peptide selon l'une quelconque des revendications 1 à 3, et une étape de détermination de la liaison du peptide avec des anticorps éventuellement présents dans l'échantillon.

11. Procédé selon la revendication 10, dans lequel l'étape de détermination de la liaison du peptide avec des anticorps éventuellement présents dans l'échantillon est réalisée par un test ELISA, par chimioluminescence ou par fixation sur un support de type micro ou macro-reseau, par immunofluorescence, par radio-immuno-marquage, par agglutination ou par hémagglutination.

12. Préparation immunogène destinée à favoriser une réponse immunitaire contre HHV-7, **caractérisée en ce qu'**elle comporte au moins un peptide selon l'une quelconque des revendications 1 à 3.

13. Utilisation d'un peptide selon l'une quelconque des revendications 1 à 3, dans la préparation d'une composition pour la détection sérologique de HHV-7, le diagnostic de HHV-7 chez un sujet, ou la stimulation d'une réponse immunitaire dirigée contre HHV-7 chez un sujet.

14. Utilisation d'un peptide selon la revendication 13, **caractérisée en ce que** ledit peptide comprend la séquence Arg-Ser-Glu-Glu-Glu-Glu.

## Claims

1. An immunogen peptide, **characterized in that** it consists of 15 to 25 amino acids and **in that** it comprises the sequence:
Ser-Val-Lys-Arg-Ser-Glu-Glu-Glu-Glu-Tyr-Val-Ala,
modified if appropriate by conservative substitution(s) which does (do) not modify the immunological reactivity of said peptide, and which conserve(s) the sequence Arg-Ser-Glu-Glu-Glu-Glu.

2. A peptide according to claim 1, corresponding to amino acids 129 to 152 of gB and defined by the peptide sequence:
Leu-Ser-Ser-Ile-Ser-Val-Lys-Arg-Ser-Glu-Glu-Glu-Glu-Tyr-Val-Ala-Tyr-His-Lys-Asp-Glu-Tyr-Val-Asn,
modified if appropriate by conservative substitution(s) which does (do) not modify the immunological reactivity of said peptide, and which conserve(s) the sequence Arg-Ser-Glu-Glu-Glu-Glu.

3. A peptide according to claim 1 or claim 2, further comprising one or more terminal amino acids heterologous to gB.

4. A kit for specific diagnosis of HHV-7, comprising a peptide according to any one of claims 1 to 3.

5. A diagnostic kit according to claim 4, **characterized in that** said peptide is fixed on a support.

6. A diagnostic kit according to claim 4 or claim 5, further comprising a detectable antibody directed against one or more classes of human immunoglobulins.

7. A diagnostic kit according to claim 6, in which the detectable antibody is radiolabelled, fluorescent or bound to an enzyme.

8. A diagnostic kit according to claim 7, in which the detectable antibody is bound to an enzyme and further comprises a substrate of said enzyme allowing a visually detectable result to be obtained.

9. A diagnostic kit according to any one of claims 4 to 8, further allowing serological diagnosis of one or more beta herpes viruses.

10. A method for serological detection of HHV-7 in a biological sample, comprising a step for bringing said biological sample into contact with a peptide according to any one of claims 1 to 3, and a step for determining binding of the peptide with antibodies which may be present in the sample.

11. A method according to claim 10, in which the step for determining binding of the peptide with antibodies which may be present in the sample is carried out by means of an ELISA test, by chemiluminescence or by binding to a micro- or macro-array type support, by immunofluorescence, by radio-immunolabelling, by agglutination or by haemagglutination.

12. An immunogenic preparation to encourage an immune response against HHV-7, **characterized in that** it comprises at least one peptide according to any one of claims 1 to 3.

13. Use of a peptide according to any one of claims 1 to 3, in the preparation of a composition for the serological detection of HHV-7, the diagnosis of HHV-7 in a subject, or to stimulate an immune response directed against HHV-7 in a subject.

14. Use of a peptide according to claim 13, **characterized in that** said peptide comprises the sequence Arg-Ser-Glu-Glu-Glu-Glu.

## Patentansprüche

1. Immunogenes Peptid, **dadurch charakterisiert, dass** es aus 15 bis 25 Aminosäuren besteht und die Sequenz
Ser-Val-Lys-Arg-Ser-Glu-Glu-Glu-Glu-Tyr-Val-Ala,
umfasst, gegebenenfalls modifiziert durch die konservative(n) Substitution(en), die die immunologische Reaktivität des Peptids nicht modifiziert (modifizieren), und das die Sequenz Arg-Ser-Glu-Glu-Glu-Glu beibehält.

2. Peptid gemäß Anspruch 1, das den Aminosäuren 129 bis 152 von gB entspricht und definiert ist durch die Peptidsequenz:
Leu-Ser-Ser-Ile-Ser-Val-Lys-Arg-Ser-Glu-Glu-Glu-Glu-Tyr-Val-Ala-Tyr-His-Lys-Asp-Glu-Tyr-Val-Asn,
gegebenenfalls modifiziert durch die konservative(n) Substitution(en), die die immunologische Reaktivität des Peptids nicht modifiziert (modifizieren), und das die Sequenz Arg-Ser-Glu-Glu-Glu-Glu beibehält.

3. Peptid gemäß Anspruch 1 oder 2, wobei das Peptid zusätzlich ein oder mehrere terminale Aminosäuren umfasst, die heterolog zu gB sind.

4. Kit, umfassend ein Peptid gemäß einem der Ansprüche 1 bis 3, der die spezifische Diagnostik von HHV-7 ermöglicht.

5. Diagnostischer Kit gemäß Anspruch 4, **dadurch charakterisiert, dass** das Peptid auf einem Träger fixiert ist.

6. Diagnostischer Kit gemäß Anspruch 4 oder 5, umfassend zusätzlich einen nachweisbaren Antikörper, der gegen eine oder mehre Klassen von humanen Immunoglobulinen gerichtet ist.

7. Diagnostischer Kit gemäß Anspruch 6, wobei der nachweisbare Antikörper radiologisch markiert, fluoreszierend oder mit einem Enzym verbunden ist.

8. Diagnostischer Kit gemäß Anspruch 7, wobei der nachweisbare Antikörper an ein Enzym gebunden ist und zusätzlich ein Substrat des Enzyms umfasst, das einen optischen Nachweis ermöglicht.

9. Diagnostischer Kit gemäß einem der Ansprüche 4 bis 8, der zusätzlich die serologische Diagnose eines oder mehrerer anderer β-Herpesviridae ermöglicht.

10. Verfahren zum serologischen Nachweis von HHV-7 in einer biologischen Probe, umfassend einen Schritt, bei dem die biologische Probe gemäß einem der Ansprüche 1 bis 3 mit einem Peptid in Kontakt gebracht wird, und einem Schritt zum Nachweis der Bindung des Peptids an einen der Antikörper, die möglicherweise in der Probe vorhanden sind.

11. Verfahren gemäß Anspruch 10, wobei der Nachweis der Bindung des Peptids an einen möglicherweise in der biologischen Probe vorhandenen Antikörper durch einen ELISA-Test, durch Chemieluminiszenz, oder durch Fixieren auf einen Mikromatrix- oder Makromatrixträger, durch Immunofluoreszenz, durch Radio-Immunmarkierung, durch Agglutination oder durch Haemagglutination durchgeführt wird.

12. Immunogene Präparation zur Begünstigung einer Immunantwort auf HHV-7, **dadurch charakterisiert, dass** die Aufbereitung mindestens ein Peptid gemäß den Ansprüchen 1 bis 3 umfasst.

13. Verwendung eines Peptids gemäß einem der Ansprüche 1 bis 3 für die Herstellung einer Zusammensetzung zum serologischen Nachweis von HHV-7, zur Diagnose von HHV-7 bei einem Individuum oder zum Auslösen einer Immunantwort auf HHV-7 bei einem Individuum.

14. Verwendung eines Peptids gemäß Anspruch 13, **dadurch charakterisiert, dass** das Peptid die Sequenz Arg-Ser-Glu-Glu-Glu-Glu umfasst.


### LISTE DE SEQUENCES

<110> UNIVERSITE PIERRE ET MARIE CURIE
<120> PEPTIDE DE LA GLYCOPROTEINE B DE L'HERPESVIRUS HUMAIN 7 UTILISABLE NOTAMMENT DANS UN TEST SEROLOGIQUE ELISA
<130> B4636A FL
<140>
   <141>
<150> 0016431
   <151> 2000-12-15
<160> 3
<170> PatentIn Ver. 2.1
<210> 1
   <211> 24
   <212> PRT
   <213> polypeptide
<400> 1
   Leu Ser Ser Ile Ser Val Lys Arg Ser Glu Glu Glu Glu Tyr Val Ala
    1               5                  10                  15
   Tyr His Lys Asp Glu Tyr Val Asn
               20
<210> 2
   <211> 6
   <212> PRT
   <213> polypeptide
<400> 2
   Arg Ser Glu Glu Glu Glu
    1               5
<210> 3
   <211> 12
   <212> PRT
   <213> polypeptide
<400> 3
   Ser Val Lys Arg Ser Glu Glu Glu Glu Tyr Val Ala
    1               5                  10